# EUROPEAN PATENT APPLICATION

(11) **EP 4 520 189 A1**
(43) Date of publication of application: **12.03.2025**
(21) Application number: 23799722.6
(22) Date of filing: 04.05.2023
(51) Int. Cl.: A23L 11/50, A23L 29/00, C12N 1/20, A23L 5/10, A23L 23/00, A23L 7/104

(54) **METHOD FOR PREPARING GRAIN TEMPEH, GRAIN TEMPEH PREPARED BY SAID METHOD, SAUCE COMPRISING PREPARED GRAIN TEMPEH, AND METHOD FOR PREPARING SAME**

(30) Priority: 06.05.2022 KR 20220056156; 03.05.2023 KR 20230058024
(71) Applicant: CJ CheilJedang Corporation, Seoul 04560 (KR)
(72) Inventor: KIM, Hee Jin, Seoul 04560 (KR); CHO, Sun A, Seoul 04560 (KR); JEON, Jin, Seoul 04560 (KR); LEE, Eun Ju, Seoul 04560 (KR)
(74) Representative: Cabinet Laurent & Charras
(86) International application number: PCT/KR2023/006158
(87) International publication number: WO 2023/214841

(57) **Abstract**

Provided are novel grain tempeh, method of producing grain tempeh, grain tempeh produced thereby, and fermented aged grain tempeh.

## Description

### TECHNICAL FIELD

### [Cross-citation with related applications]

This application claims priority to Korean Patent Application No. 10-2022-0056156, filed on May 6, 2022, and Korean Patent Application No. 10-2023-0058024, filed on May 3, 2023, the disclosures of which are incorporated herein by reference.

### [TECHNICAL FIELD]

The present disclosure relates to a method of producing grain tempeh, grain tempeh produced by the method, a sauce including the grain tempeh, and a method of producing the sauce.

### BACKGROUND

Tempeh is a traditional Indonesian food made from fermented beans, and generally, the production method thereof includes boiling and peeling beans, inoculating Rhizopus strains mainly, and carrying out fermentation. Tempeh is made from beans like tofu, but its nutritional characteristics and texture are different from those of tofu. For example, its shape is similar to that of tofu, but its taste is similar to that of mushrooms. In the fermentation process for producing tempeh, as Rhizopus strains grow, the mycelium envelops the beans and clumps together to make a hard tissue, and thus, tempeh is also used as a meat substitute.

In Indonesia, tempeh is an everyday food ingredient which is cut and fried or sauteed. In addition, tempeh contains abundant amino acids and vitamins, and in particular, usually contains calcium in 2/3 of a cup of milk.

Currently, since tempeh is produced by a conventional method such as handwork rather than a mass production method and is produced in the form of being wrapped in banana leaf or plastic during the production, there is a possibility of cross-contamination in the production process and the production is not appropriate for mass production.

In addition, tempeh is being produced using beans such as soybeans, black beans, and chick peas as a raw material, but from the point of view of nutritional enhancement of tempeh, and availability and expandability to be applied to the preferences of different races, the need for development of tempeh produced using a raw material other than beans is being constantly demanded.

Therefore, in order to use the tempeh having a nutritionally superior value in a variety of ways, the present inventors made diligent research efforts to develop a method of producing tempeh using grains as a raw material and a sauce using grain tempeh produced in this manner, and as a result, completed the present disclosure.

### DISCLOSURE OF THE INVENTION

### TECHNICAL PROBLEM

An object of the present disclosure provides grain tempeh produced by a method of producing grain tempeh.

Another object of the present disclosure provides aged grain tempeh having an excellent flavor by aging grain tempeh.

Still another object of the present disclosure provides a sauce including grain tempeh or aged grain tempeh.

### TECHNICAL SOLUTION

In order to achieve the above objects,

An aspect of the present disclosure provides a method of producing grain tempeh includes: (a) steaming grains; (b) inoculating a tempeh fungus into the steamed grains; and (c) fermenting the steamed grains inoculated with the tempeh fungus.

Another aspect of the present disclosure provides a method of producing aged grain tempeh includes aging the grain tempeh.

Still another aspect of the present disclosure provides a sauce includes grain tempeh and aged grain tempeh.

Hereinafter, the present disclosure will be described in detail.

### 1. Method of producing grain tempeh

An aspect of the present disclosure provides a method of producing grain tempeh including: (a) steaming grains; (b) inoculating a tempeh fungus into the steamed grains; and (c) fermenting the steamed grains inoculated with the tempeh fungus.

The method of producing grain tempeh includes (a) steaming grains.

In the present disclosure, grains collectively refer to grains obtained from plants, which are substances serving as human food, and for example, may be wheat, wheat rice, rye, rice (white rice, brown rice, and black rice), barley, foxtail millet, oats, proso millet, sorghum, corn, and the like. The grains may be in an original grain form, a dried form of original grains, or a powder form thereof.

The step of steaming grains may be performed by a continuous steaming method or a steam pressurization method.

The continuous steaming refers to continuous steaming of grains in a state of being spread on a support by steam, and for example, may be placing the grains on a moving conveyor belt and spraying hot water or steam from the upper or lower portion of the belt to continuously steam a large amount of grains within a short time. Since the continuous steaming may adjust a steaming time to a conveyor belt speed while adding water to grains by steam direct injection and may not clump grains having a powder form or a particle size during steaming so as to alphaize the grains evenly, it has an effect of improving production efficiency.

In addition, the steaming step may be performed by a steam pressurization method. The steam pressurization method may use saturated steam in which water and steam are in equilibrium, and since the saturated steam emits heat without taking away moisture of grains, grains may be rapidly steamed using saturated steam. When steaming is performed by the pressurization method of saturated steam, the pressure may be 0.5 kgf/cm² to 3 kgf/cm², 0.6 kgf/cm² to 2.7 kgf/cm², 0.7 kgf/cm² to 2.5 kgf/cm², 0.8 kgf/cm² to 2.3 kgf/cm², or 1.0 kgf/cm² to 2.0 kgf/cm². In addition, the saturated steam may be at a temperature of 60°C to 120°C, 70°C to 110°C, or 80°C to 100°C. Herein, the steaming by a steam pressurization method may use a pressurization-type steamer, for example, a rotary pressurization-type steamer (NK-type steamer) or a batch pressurization steamer.

The grains steamed after the steaming step may be stirred or crushed into a smaller size for the convenience of tempeh fungus inoculation and tempeh molding before inoculating a tempeh fungus.

The method of producing grain tempeh includes (b) inoculating a tempeh fungus into the steamed grains.

The tempeh fungus is the strain of the genus Rhizopus, and for example, may be *Rhizopus oligosporus.* The tempeh fungus is an edible mushroom fungus which grows naturally on banana leaves, contains a large amount of superdismutase which decomposes lipids which are not good for the human body, and has an activity of activating intestinal immunity to inhibit malignant Escherichia coli and the like. The inoculation of the tempeh fungus may be addition of a tempeh starter including the tempeh fungus. The tempeh starter may be obtained by artificially inoculating purely cultured Rhizopus strains into steamed grains and culturing the grains, and for example, may be in a mixed form of rice flour and a tempeh fungus or in a seed koji form in which a Rhizopus fungus is sporulated in rice flour, and may be a mixture of 90-99.9 wt% of rice flour and 0.1-10 wt% of a tempeh fungus, 95-99.9 wt% of rice flour and 0.1-5 wt% of a tempeh fungus, or 90-99.9 wt% of rice flour and 0.1-10 wt% of a tempeh fungus. In step (b), the tempeh starter may be added at a rate of 1 wt% to 10 wt%, 1 wt% to 9 wt%, 1 wt% to 8 wt%, 2 wt% to 8 wt%, 3 wt% to 7 wt%, or 4 wt% to 6 wt% with respect to the weight of raw material grains to the steamed grains. The content of the tempeh fungus inoculated into the steamed beans may be 0.01 wt% to 0.1 wt%, 0.01 wt% to 0.09 wt%, 0.01 wt% to 0.08 wt%, 0.02 wt% to 0.08 wt%, 0.03 wt% to 0.07 wt%, or 0.04 wt% to 0.06 wt% with respect to the total weight of the steamed grains.

As the inoculation amount of the tempeh fungus increases, the tempeh fungus multiplies predominantly, and thus, the growth of pathogenic microorganisms including common microorganisms or food poisoning bacteria is inhibited and tempeh having excellent quality may be produced.

The method of producing grain tempeh may further include adding an acid to the steamed grains in step (b).

The acid refers to a material which is acidic and tastes sour when it is dissolved in water, and a usable acid may be, for example, vinegar, citric acid, lactic acid, lemon juice, malic acid, gluconic acid, and the like, but is not limited thereto, and is not limited as long as it is an edible acid. By adding the acid, acidity appropriate for culturing a tempeh fungus may be maintained and the tempeh fungus grows predominantly, so that the growth of other common microorganisms, fungi, and pathogenic microorganisms is inhibited. The acidity may be acidity at which the growth of a tempeh fungus is not inhibited and the growth of other microorganisms, for example, pathogenic microorganisms or fungi is not inhibited. The acidity (%) of the acid refers to the content (g) of the acid included in 100 mL of an acid solution, and the acidity may be in a range selected from any one lower limit selected from the group consisting of 5%, 6%, 7%, 9%, and 10%; and any one upper limit selected from the group consisting of 12%, 13%, 15%, 16%, 17%, 18%, 19%, 20%, and 25%, and specifically, the acidity may be in a range of 5% to 20%, 7 to 19%, 9 to 15%, 10 to 13%, or 10 to 12%. The acid may be added when the product temperature of the steamed beans is 50°C or lower.

In the step of adding an acid to the steamed grains in step (b), the acid may be inoculated into the steamed grains at a rate of 1 wt% to 10 wt%, 1 wt% to 9 wt%, 1 wt% to 8 wt%, 2 wt% to 8 wt%, 3 wt% to 7 wt%, or 4 wt% to 6 wt% with respect to the weight of raw material grains.

In an exemplary embodiment, the adding of an acid to the steamed grains in step (b) may be performed before, after, or at the step of inoculating a tempeh fungus into the steamed grains.

The method of producing grain tempeh includes (c) fermenting the steamed grains inoculated with the tempeh fungus.

The step of fermenting the steamed grains inoculated with the tempeh fungus may refer to a step of performing culture so that the tempeh fungus may multiply in grains inoculated with the tempeh fungus. The tempeh fungus culture may be performed at an appropriate temperature and humidity. For example, the fermentation may be performed in a temperature range selected from any one lower limit selected from the group consisting of 10°C, 15°C, 18°C, 20°C, 22°C, 24°C, 25°C, 26°C, 28°C, and 30°C and any one upper limit selected from the group consisting of 30°C, 32°C, 33°C, 35°C, 38°C, 40°C, 43°C, 45°C, and 50°C. For example, it may be performed at 15°C to 45°C, 18°C to 43°C, 20°C to 40°C, 22°C to 38°C, 25°C to 35°C.

In addition, a fermentation time may be 10 hours to 15 days, 15 hours to 15 days, 20 hours to 15 days, 1 day to 15 days, 1 day to 14 days, 1 day to 13 days, 1 day to 12 days, 1 day to 11 days, 1 day to 10 days, 1 day to 9 days, 1 day to 8 days, 1 day to 7 days, 1 day to 6 days, 1 day to 5 days, 1 day to 4 days, 1 day to 2 days, or 2 days to 4 days. In addition, a humidity during the fermentation may be 40% to 90%, 40% to 85%, 40% to 80%, 40% to 70%, 50% to 70%, 50% to 80%, 60% to 80%, 55% to 65%, or 65% to 75%. When the fermentation is performed in the above conditions, the growth of a tempeh fungus predominates and microbial growth is inhibited, thereby improving the storage stability of tempeh excellently. In addition, the tempeh produced in the fermentation conditions has uniformly improved maturation and good quality, and thus, is suitable for mass production.

The method of producing grain tempeh may further include cooling the steamed grains. The cooling step may be performed in a product temperature range having any one lower limit selected from the group consisting of 10°C, 15°C, 18°C, 20°C, 22°C, 24°C, 26°C, 28°C, and 30°C and any one upper limit selected from the group consisting of 30°C, 32°C, 33°C, 35°C, 38°C, 40°C, 45°C, and 50°C so that the temperature is appropriate for fermentation. By cooling, oversteaming by residual heat is prevented and an environment in which the tempeh fungus grows well may be created.

In the step of (c) fermenting the steamed grains inoculated with the tempeh fungus, the grains inoculated with the tempeh fungus may be put in a perforated container made of polyethylene (PE) and then be fermented. When the container made of polyethylene is used, heating may be easily adjusted, grain tempeh having good quality which is fermented well by a tempeh fungus may be produced, and grain tempeh which is evenly fermented may be produced. In addition, the container may be perforated. Since air may circulate by perforation in the container, the container has high oxygen permeability and may perform fermentation by a tempeh fungus effectively.

The production method may further include molding the grains inoculated with the tempeh fungus, in the step of (b) inoculating a tempeh fungus into the steamed grains. In the specification of the present disclosure, the steamed grains inoculated with the tempeh fungus may be referred to as "tempeh" even before fermenting the grains. The tempeh (steamed beans inoculated with the tempeh fungus) may be molded so that a height is 2 cm to 5 cm or 3 cm to 4.5 cm. When the height of tempeh is in the above range, the growth of a tempeh fungus is facilitated so that the mycelium extends evenly to a deep part so that fermentation is well done, and thus, tempeh having excellent quality which is hard, has good texture, and has a low microbial level may be efficiently produced.

When the method as above is used, grain tempeh produced using various grains may be produced without beans, and the growth of microorganisms may be inhibited to produce grain tempeh having excellent storage and distribution stability.

Another aspect of the present disclosure provides grain tempeh produced by the production method.

The grain tempeh produced by the production method may have a moisture content of 20 to 45%, 25 to 40%, 26 to 39%, 27 to 38%, 28 to 39%, 29 to 38%, or 30 to 36% with respect to the total weight of tempeh. When the moisture content included in tempeh is in the range described above, microbial stability is excellent.

In the grain tempeh produced by the production method, the growth of a tempeh fungus predominates to inhibit microbial growth, and thus, storage stability is excellent.

Tempeh is an Indonesian traditional fermented food and has many live fungi, and those fungi may include harmful bacteria which harm the human body, such as food poisoning bacteria. Therefore, in order to secure distribution stability without losing the advantage as fermented food, such as a flavor caused by a fermented product by an enzyme and a tempeh fungus, contamination with harmful bacteria should be decreased.

The microorganisms may be *Bacillus cereus,* common bacteria, fungi, and the like. The *Bacillus cereus* is a kind of soil bacterium and is widely distributed in nature such as dust, sewage, and streams including a human living environment, and is called food poisoning bacteria, as bacteria which cause decay of plants and animals and disease in human and animals. Since the growth of a tempeh fungus predominates in the grain tempeh produced by the production method, Bacillus cereus bacteria may be detected at 10⁵ CFU/g or less, 10⁴ CFU/g or less, 10³ CFU/g or less, or 10² CFU/g or less. In the grain tempeh produced by the production method, the common bacterial count may be detected at 2×10⁸ CFU/g or less.

In an example, when a tempeh fungus starter is added at 3 to 6 wt% with respect to the weight of the raw material grains, the growth of a tempeh fungus predominates, and thus, the *Bacillus cereus* bacteria may be detected at 10³ CFU/g or less and common bacteria may be detected at 10⁹ CFU/g or less.

In an example, when a tempeh fermentation temperature is 25°C to 35°C, the Bacillus cereus bacteria may be detected at 10³ CFU/g or less.

In an example, when a tempeh fermentation humidity is 65% to 75%, common bacteria may be detected at 10⁹ CFU/g or less.

In an experimental example, the Bacillus cereus bacteria detected in the grain tempeh produced by the production method of the present disclosure are 10³ CFU/g or less, which satisfies the microbial safety standards according to the domestic food standards and specifications. In addition, since the grain tempeh has more inhibited growth of microorganisms than tempeh made from beans, it has better distribution stability than bean tempeh, and may be applied to various foods and used.

The grain tempeh of the present disclosure and a method of producing grain tempeh provide tempeh having a new concept such as tempeh produced with grains and a method of producing the tempeh, thereby expanding the scope of use of tempeh, and thus, for example, are used as a raw material or an additive in the production of sauces or pastes.

### 2. Method of producing aged grain tempeh

Another aspect of the present disclosure provides a method of producing aged grain tempeh including: (a) producing grain tempeh by the method of producing grain tempeh described above, and (b) an aging step of aging a mixture in which at least one or more of saline and brine are added to the grain tempeh produced above.

In step (a) of the method of producing aged grain tempeh, a step of producing grain tempeh is producing grain tempeh by the method of producing grain tempeh described above, and as described above, may include steaming grains; inoculating a tempeh fungus into the steamed grains; and fermenting the steamed beans inoculated with the tempeh fungus. Since the details of the method of producing grain tempeh are the same as those in the method of producing grain tempeh described above, it is cited and the description is not duplicated.

The method of producing aged grain tempeh includes (b) an aging step of aging a mixture in which at least one or more of saline and brine are added to the grain tempeh.

The aging is fermenting a grain tempeh is fermented according to a fermentation flavor to be desired to produce the aged tempeh. The aging may be performed at, for example, at 10°C to 50°C, and for example, may be performed at 15°C to 45°C, 20°C to 40°C, 25°C to 45°C, 25°C to 35°C, 35°C to 45°C, or 25°C to 35°C. The aging may be performed at, for example, 20°C, 30°C, or 40°C. In addition, the aging may include fermentation for 1 day to 30 days, and for example, may include fermentation for 1 day to 25 days, 1 day to 20 days, 1 day to 15 days, 1 day to 14 days, 3 days to 14 days, or 3 days to 15 days. When the aging temperature and/or the aging days are within the range, the nutrients or flavor of the aged tempeh produced may be improved excellently, and the microbial growth in the produced aged tempeh may be inhibited to improve the storge stability of food, but the present disclosure is not limited thereto.

The aging step may include further adding at least one or more of grains and steamed grains to the mixture. Since grains may be the same as those described in the method of producing grain tempeh, the description of the grains is cited and is not duplicated.

In a specific example, grains or steamed grains are added to aged grain tempeh as extra rice to adjust an aging degree. The grains or the steamed grains may be added at 1 wt% or more, 2 wt% or more, 3 wt% or more, 4 wt% or more, 5 wt% or more, 6 wt% or more, 7 wt% or more, 8 wt% or more, 10 wt% or more, 12 wt% or more, 14 wt% or more, 16 wt% or more, 18 wt% or more, or 20 wt% or more with respect to the total weight of the mixture, but are not limited thereto, and may be appropriately adjusted depending on the aging degree or flavor to be desired.

In the aging step, a moisture content of the grain tempeh or a mixture of the grain tempeh and saline, brine, grains, or steamed grain may be 40 to 70 wt%, 45 to 65 wt%, or 50 to 60 wt%. When the moisture content of the mixture is in the above range, aging is well performed. In addition, in the aging step, at least one or more of the saline or brine may be added so that a salt concentration is 5 to 15 wt%, for example, 5 to 10 wt%, 5 to 7 wt%, or 7 to 9 wt% with respect to the total weight of the mixture added. When the moisture content and/or the salt concentration in the mixture is within the range, the nutrients of the produced aged tempeh may be improved, and the microbial growth in the produced aged grain tempeh is inhibited to improve storage stability of food.

In an example, when the moisture content of the mixture is 50% or 60%, the content of amino-type nitrogen is effectively improved to increase an aging degree.

In addition, when steamed grains are mixed with the grain tempeh, the aging degree of grain tempeh may be adjusted according to the purpose.

In an example, the steamed grain in the grain tempeh may be added at 1 wt% to 50 wt%, 2 wt% to 45 wt%, 3 wt% to 40 wt%, 5 wt% to 35 wt%, or 5 wt% to 30 wt% with respect to the total weight of tempeh, thereby adjusting the aging degree of the aged tempeh. By adding the steamed grains as extra rice to adjust an aging degree, aged products having various flavors and sauces including them may be produced.

In addition, the aging step may further include stirring the mixture. When the mixture is stirred, tempeh may be effectively fermented so that the aging of the aged grain tempeh may be well performed. The stirring may be performed by a stirrer, for example, an impeller, and a stirring speed may be 10 to 500 rpm, 25 to 450 rpm, 30 to 400 rpm, specifically 50 to 300 rpm. The stirring may be performed for 1 minute to 1 hours once a day, twice a day, or continuously during an aging period.

The stirring may be performed at a speed of 50 to 300 rpm and may be continuously performed for 1 minute to 30 minutes during the aging period or during the aging period.

In an example, as a result of evaluating the aging degree with the content of amino-type nitrogen by different aging methods such as static fermentation or stirring fermentation in the aging of the grain tempeh, it was confirmed that the aging degree of the aged product which has been aged by stirring is improved.

In addition, the aging degree may be adjusted by repeating the aging step once or more, twice or more, or three times or more.

Another aspect of the present disclosure provides aged grain tempeh produced by the production method.

The content of amino-type nitrogen included in the aged tempeh may be 5 mg% or more and 1200 mg% or less, and specifically, in a range selected from a lower limit of 40 mg%, 80 mg%, 100 mg%, 150 mg%, 200 mg%, 250 mg%, 300 mg%, or 400 mg% and an upper limit of 1200 mg%, 1100 mg% or less, or 1000 mg%. More specifically, it may be 40 mg% to 1200 mg%, 100 mg% to 1100 mg%, or 200 mg% to 1000 mg%.

The amino-type nitrogen is an indicator of an aging degree which shows the content of amino acid produced by decomposition of protein by an enzyme produced by microorganisms by aging, and since its content is important for the taste of soy sauces such as umami, it is generally a management standard of soy sauce. The aged grain tempeh of the present disclosure may be produced into an aged product having a desired aging degree by adjusting the type of raw material grains, an aging period, an aging temperature, and a rotation speed during stirring, depending on the type of sauces to be produced.

For example, when red pepper pastes are to be produced with the aged grain tempeh, aged tempeh produced to have an aging degree of 100 to 300 mg% may be used, when ssamjang is to be produced, aged tempeh produced to have an aging degree of 220 mg% or more may be used, and when soybean sauce is to be produced, aged tempeh produced to have an aging degree of 400 mg% or more may be used and aged. The aging degree may be appropriately adjusted by the type of raw material grains, an aging period, an aging temperature, an aging method, or an inoculation amount of the tempeh fungus.

When the method of producing aged grain tempeh according to the present disclosure is used, a raw material of a new sauce having an enhanced flavor as compared with raw material grains or grain tempeh before aging may be provided.

### 3. Sauce including grain tempeh or aged grain tempeh

Another aspect of the present disclosure provides a sauce including grain tempeh produced by the production method and the above grain tempeh.

The sauce including the aged grain tempeh of the present disclosure may be used as a raw material for producing a new sauce beyond a simple food material, and thus, the field of application is broadened to increase tempeh utility.

The sauce including tempeh may include a content of 1 to 99 wt%, for example, 1 to 90 wt%, 1 to 80 wt%, 1 to 70 wt%, 1 to 60 wt%, 1 to 50 wt%, 1 to 40 wt%, 1 to 35 wt%, 1 to 30 wt%, 1 to 10 wt%, or 3 to 27 wt% of the tempeh.

The sauce including tempeh may be the tempeh or aged tempeh simply diluted in water, but for enhancing the flavor of the sauce including tempeh, one or more additive selected from the group consisting of spice plants, processed spices, pastes, sugars, flavor enhancers, salt, vinegar, nuts, composite seasoned foods, and fermented bean foods are mixed with the aged tempeh, and thus, it may further include one or more selected from the group consisting of spice plants, processed spices, pastes, sugars, flavor enhancers, salt, vinegar, nuts, composite seasoned foods, and fermented bean foods.

The "spice plant" is a plant to add for adding the taste and flavor of a sauce, and though it is not limited thereto, it may include chili pepper, garlic, ginger, black pepper, onion, green onion, shallot, and the like.

The "processed spice" is obtained by simply processing leaves, stems, fruits, roots, or the like of spice plants or mix-processing food additives, and is used for increasing other food flavors. The processed spice is not limited, but may include, for example, natural spices such as red pepper powder, black pepper powder, cinnamon powder, oregano hall, and rosemary, and spice preparations such as mustard, curry, and tomato ketchup.

The "composite seasoned food" is processed into powder, granules, or a solid shape by mixing food with sugars, salt, spices, protein hydrolysate, yeast or its extracts, or food additives, and for example, a chili pepper seasoning may be used, and the chili pepper seasoning is a paste made by mixing chili pepper powder, onion, garlic, water, salt, and the like.

The "bean fermented food" is a product obtained by mixing and fermenting beans, specifically soybeans or soybean cake and sugar, and is a food having liquid physical properties having a sugar content of 30% or more.

The "pastes" are obtained by manufacturing/processing aspergillus culture in animal/vegetable raw materials or fermented/aged mixture of fermented soybean lump as a raw material, salt, and the like, and though they are not limited, for example, they include Korean fermented soybean lump, modified soybean lump, Korean soy sauce, brewed soy sauce, acid-decomposed soy sauce, enzyme-decomposed soy sauce, mixed soy sauce, Korean soybean paste, soybean paste, chili pepper paste, chunjang, fast-fermented bean paste, mixed soybean paste, and the like.

The "sugars" represent sugars obtained by processing starch raw materials or sugar solution, sugar syrups, oligosaccharides, glucose, fructose, taffy, or sugar products processed therefrom.

The "flavor enhancer" is for enhancing the taste or flavor of food, and may include, for example, sodium glutamate and the like.

The "nuts" are not limited as long as they are edible nuts, and may include, for example, peanuts, almonds, walnuts, macadamia, hazelnuts, pine nuts, acorns, nutmegs, Brazil nuts, cashews, coffee beans, cocoa beans, pistachios, pecans, sunflower seeds, and the like.

In a specific example, the tempeh-containing sauce may further include at least one or more of chili pepper powder, chili pepper, salt, soy sauce, tomato ketchup, and other spice plants, processed spices, sugars, flavor enhancers, composite seasonings, and nuts.

The sauce including tempeh may not include additives other than the components described above, or may further include other additives. When other additives are further included, such additives may include preservatives and/or excipient acceptable for food.

The preservatives acceptable for food are not limited, and may include, for example, sorbic acids, benzoic acids, dehydroacetic acids, paraoxybenzoic acids, propionic acids, and the like. The preserves may use, for example, a salt form such as potassium sorbate, calcium sorbate, sodium benzoate, sodium propionate, calcium propionate, and sodium paraoxybenzoate.

The excipients acceptable for food is not limited, and may be, for example, at least one selected from the group consisting of crosslinked sodium carboxymethylcellulose, gum ghatti, persimmon colorant, licorice extract, formic acid, geranyl formate, citronellyl formate, isoamyl formate, gum resin, geraniol, crystalline cellulose, cinnamic acid, methyl cinnamate, ethyl cinnamate, cinnamic aldehyde, cinnamon alcohol, kaoliang colorant, benzoyl peroxide, hydrogen peroxide, acetic acid peroxide, ammonium persulfate, guar gum, disodium 5'-guanylate, citric acid, manganese citrate, trisodium citrate, sodium ferrous citrate, iron citrate, ammonium iron citrate, potassium citrate, calcium citrate, magnesium silicate, calcium silicate, silicon resin, diatomaceous earth, gluconic acid, sodium gluconate, copper gluconate, magnesium gluconate, manganese gluconate, zinc gluconate, iron gluconate, potassium gluconate, calcium gluconate, glutaminase, butyric acid, butyl butyrate, ethyl butyrate, isoamyl butyrate, neotame, nisin, nicotinic acid, nickel, nicotinamide, dextranase, dextran, sodium lauryl sulfate, lactase, lactoferrin concentrate, lactitol, lecithin, rosin, locust bean gum, rutin, linalool, mannitol, maltol, D-maltitol, sodium metasilicate, sodium metaphosphate, potassium metadiate, sodium metabisulfite, potassium metabisulfite, sodium methoxide, anhydrous sulfurous acid, myristic acid, microfibrillar cellulose, vanillin, white clay, betaine, bentonite, powdered cellulose, sodium fluoride, biotin, vitamins, glacial acetic acid, DL-malic acid, sodium saccharin, saffron colorant, acid clay, acidic sodium sulfite, acidic sodium aluminum phosphate, acidic sodium pyrophosphate, acidic calcium pyrophosphate, magnesium oxide, zinc oxide, calcium oxide, methyl salicylate, ferric oxide, fiber wax, sodium hydroxide, potassium hydroxide, calcium hydroxide, magnesium hydroxide, sucralose, shellac, steviol glycosides, stearic acid, stearates, food coloring, benzoic acid, benzoates, alginic acid and alginate, inositol, silicon dioxide, chlorine dioxide, carbon dioxide, titanium dioxide, xanthan gum, starch, modified starch, lactic acid and lactates, gelatin, gellan gum, seed koji, carnauba wax, carrageenan, gum karaya, carotene, carboxymethylcellulose sodium, carboxymethylcellulose calcium, carboxymethyl starch sodium, casein and caseinate salts, chitosan, chitin, tara gum, tamarind gum, taurine, tannic acid, palmitic acid, phenyl ethyl acetate, phenyl isobutyl acetate, pectin, pepsin, hydroxypropyl methyl cellulose, hydroxypropyl cellulose, hyaluronic acid, and yeast extract.

### ADVANTAGEOUS EFFECTS

By the method of producing grain tempeh of the present disclosure, grain tempeh using grains as a raw material may be produced. Various grains may be used to produce grain tempeh of a new material. The method of producing grain tempeh of the present disclosure is a less possibility of cross contamination during production, and the quality of tempeh may be maintained uniformly to allow mass production. The method of producing tempeh of the present disclosure is an optimal production method according to a grain raw material considering the properties of a tempeh strain, and may efficiently mass produce grain tempeh which is hard and has good quality, and the grain tempeh produced thereby has a low microbial level, and thus, has excellent distribution stability.

In addition, the range of food to which the grain tempeh is applied is wider than that the bean tempeh is applied, and the microbial growth is decreased as compared with the bean tempeh, and thus, the grain tempeh may be used as a raw material of food having excellent distribution stability.

The present disclosure provides the grain tempeh having a novel concept and produces a sauce including aged tempeh, thereby using tempeh as a raw material for producing a novel sauce beyond conventionally using tempeh only as a food material to further broaden the field of application of tempeh and increase tempeh utility.

However, the effect of the present disclosure is not limited to the effects mentioned above, and other effects which are not mentioned herein may be clearly understood by a person skilled in the art from the following description.

### BRIEF DESCRIPTION OF THE DRAWINGS

FIG. 1 shows a method of producing wheat tempeh according to an exemplary embodiment of the present disclosure.
FIG. 2 shows a method of producing aged wheat tempeh by aging the wheat tempeh produced according to an exemplary embodiment of the present disclosure.
FIG. 3 shows a difference in microbial levels of wheat tempeh depending on the added amount of a tempeh starter.
IG. 4 shows a difference in microbial levels of wheat tempeh depending on the fermentation condition (humidity) of tempeh.
FIG. 5 shows the quality of tempeh produced by performing tempeh fermentation in a condition of a relative humidity of 30%.
FIG. 6 shows a difference in microbial levels of wheat tempeh depending on the fermentation condition (temperature) of tempeh.

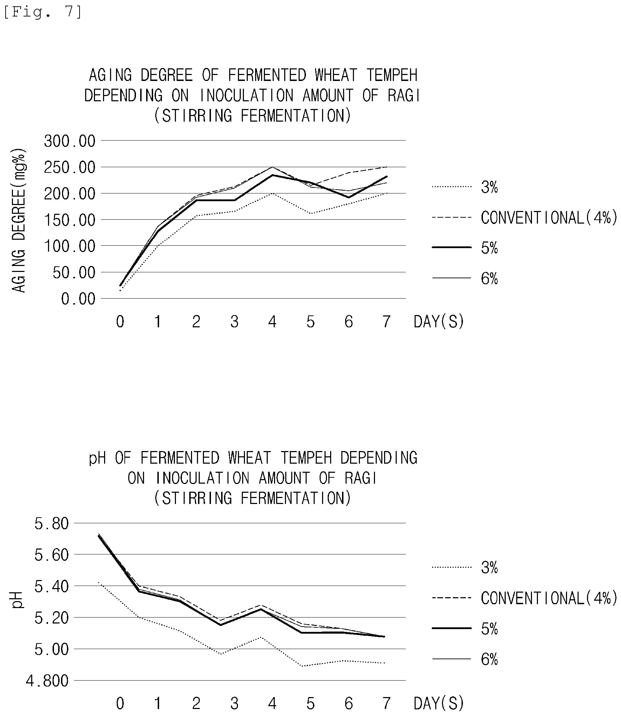

FIG. 7 shows results of comparison of aging degree and pH of aged products obtained by aging wheat tempeh produced with different inoculation amounts of a tempeh fungus.
FIG. 8 shows results of comparison of aging degree of aged products depending on whether wheat tempeh is stirred (stirred, static) during aging.
FIG. 9 shows results of comparison of aging degree and pH of aged products obtained by aging wheat tempeh produced with different fermentation condition (relative humidity) .
FIG 10 shows results of comparison of aging degree and pH of aged products obtained by aging wheat tempeh produced with different fermentation condition (temperature).
FIG. 11 shows results of comparison of aging degree depending on a moisture content of the mixtures when steamed wheat rice (extra rice) was mixed with wheat tempeh to produce aged mixture of wheat tempeh and wheat rice.
FIG. 12 shows results of comparison of aging degree of wheat aged tempeh depending on a content of steamed wheat rice (extract rice) added to wheat tempeh.
FIG. 13 shows results of comparison of aging degree and pH of aged grain tempeh depending on the type of raw material grains.

### MODE FOR CARRYING OUT THE INVENTION

Hereinafter, the present disclosure will be described in detail by the examples.

However, the following examples only specifically illustrate the present disclosure, and the content of the present disclosure is not limited by the examples.

### [Example 1] Production of wheat tempeh

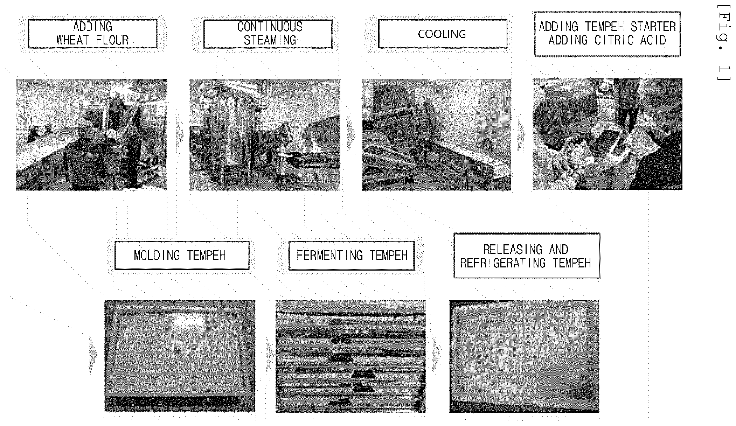

As shown in FIG. 1, raw material wheat flour having a moisture content of 13±2% was placed on a conveyor belt, and continuously steamed using steam spraying while moving the conveyor belt. Thereafter, steamed wheat flour was passed through a cooler to cool a product temperature of 30°C.

When the wheat flour was continuously steamed as described above, wheat flour did not clump to improve production efficiency. In addition, oversteaming by residual heat was prevented by a process of cooling after steaming and powder clumping was prevented to improve production efficiency.

Next, a tempeh starter including 99 wt% of rice flour and 1 wt% of Rhizopus was inoculated into the steamed and cooled wheat flour at 4 wt% with respect to the weight of raw material wheat flour, 4 wt% of citric acid with respect to the weight of the raw material wheat flour was added, and stirring was performed for 8 minutes to produce a mixture. The mixture prepared above was put into a perforated polyethylene tray and molded to have a height of 3 cm. The polyethylene tray having the molded mixture was covered with a perforated board (lead) and transferred to a fermentation room. Fermentation was performed for 44 hours to 48 hours in a fermentation room in which air was circulated and a temperature of 27°C and a humidity of 60% were maintained, thereby producing wheat tempeh. The produced wheat tempeh was kept refrigerated at 5°C for 7 days.

### [Example 2] Production of aged wheat tempeh

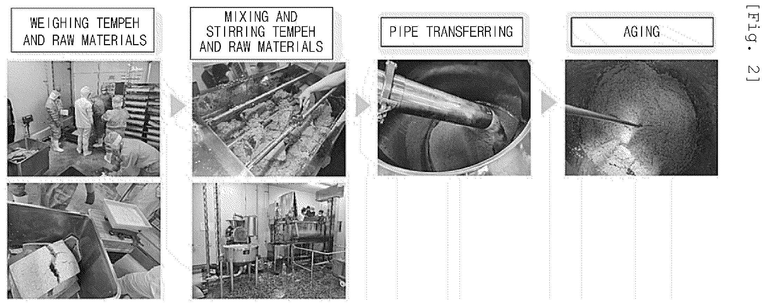

As shown in FIG. 2, brine having a salinity of 7% was added to the wheat tempeh produced in Example 1 so that a moisture content was adjusted to about 60%, and aging was performed while performing stirring at room temperature at a speed of 50 to 300 rpm, thereby producing aged wheat tempeh.

Wheat tempeh produced by changing an added amount of tempeh starter, fermentation humidity, and fermentation temperature in the wheat tempeh production was further aged to produce an aged product, and the amino-type nitrogen content and pH of the produced aged product were measured, thereby evaluating the effect of the production conditions during the wheat tempeh production on the aging degree of the aged product. The results are shown in the following Experimental Example 2.

In addition, steamed wheat rice and brine were added to the wheat tempeh produced in Example 3 to prepare a mixture, and the prepared mixture was stirred and aged to produce an aged product (aged mixture) of wheat tempeh and wheat rice.

### [Example 3] Production of rice tempeh

Raw material rice flour having a moisture content of 13±2% was placed on a conveyor belt and continuously steamed using steam spraying while moving the conveyor belt, or transferred to a rotatable NK steam tank and steamed by pressurizing saturated steam of 1.0 to 2.0 kgf/cm² at about 90°C for 2 minutes and 30 minutes. Thereafter, steamed rice powder was passed through a cooler to cool a product temperature of 30°C.

Next, a tempeh starter including 99 wt% of rice flour and 1 wt% of Rhizopus was inoculated into the steamed and cooled rice flour at 4 wt% with respect to the weight of raw material rice flour, 4 wt% of citric acid with respect to the weight of the raw material rice flour was added, and stirring was performed for 8 minutes to produce a mixture. The mixture prepared above was put into a perforated polyethylene tray and molded to have a height of 3 cm. The polyethylene tray having the molded mixture was covered with a perforated board (lead) and transferred to a fermentation room. Fermentation was performed for 44 hours to 48 hours in a fermentation room in which air was circulated and a temperature of 27°C and a humidity of 60% were maintained, thereby producing rice tempeh. The produced rice tempeh was kept refrigerated at 5°C for 7 days.

### [Experimental Example 1] Evaluation of quality depending on production method of grain tempeh

### 1-1. Evaluation of quality of wheat tempeh depending on inoculation amount of tempeh fungus

Wheat tempeh was produced in the same manner as in Example 2, except that the added amount of a tempeh starter was changed to 3 wt% to 6 wt% with respect to the total weight of the wheat flour. *Bacillus cereus* which is food poisoning bacteria was counted for the produced wheat tempeh and microbial levels were compared.

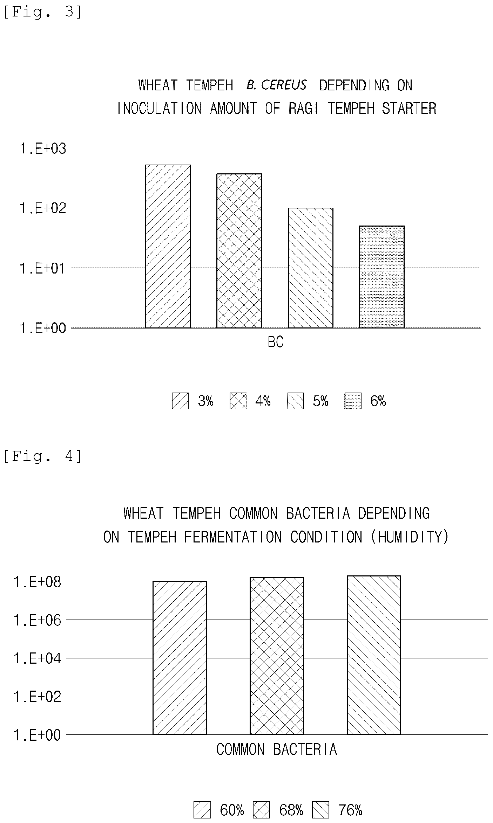

Specifically, a specimen of the wheat tempeh was smeared on a selected medium and the bacillus cereus was counted. As a result, as shown in the following Table 1 and FIG. 3, it was confirmed that as the inoculation amount of a tempeh fungus (amount of tempeh starter added) was increased, the growth of a tempeh fungus predominated, so that *Bacillus cereus* count was decreased.

**[Table 1]**

| Amount (%) of tempeh starter added to wheat flour | B. Cereus (CFU/g) |
|---|---|
| 3 wt% | 5.E+02 |
| 4 wt% | 4.E+02 |
| 5 wt% | 1. E+02 |
| 6 wt% | 5.E+01 |

### 1-2. Evaluation of quality of wheat tempeh depending on tempeh fermentation humidity

Wheat tempeh was produced in the same manner as in Example 1, except that the fermentation humidity during tempeh fermentation was changed to 30%, 60%, 68%, or 76%. The specimen of the wheat tempeh produced was smeared on a standard agar medium, common bacteria were counted, and microbial levels were compared.

As a result, as shown in the following Table 2 and FIG. 4, it was confirmed that as the humidity was decreased in a relative humidity range of 60 to 78%, the growth of a tempeh fungus predominated, so that common bacterial count was decreased, but the growth of a tempeh fungus was all smoothly done at a relative humidity of 60 to 78% to complete tempeh which was hard and had good quality.

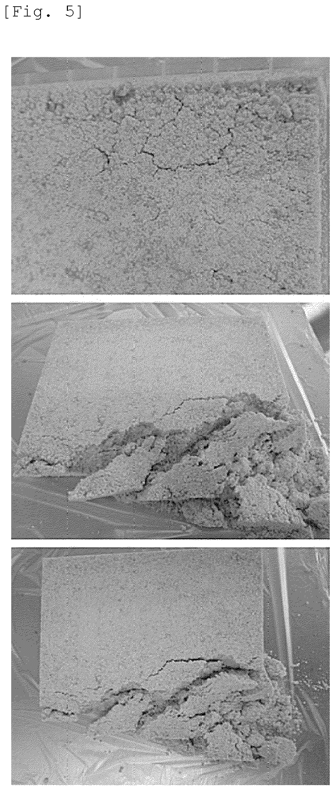

However, in the wheat tempeh produced in the conditions of a relative humidity of 30%, as shown in FIG. 5, a tempeh fungus did not grow well, and thus, white hyphae of a tempeh fungus was not spread to a deep part even visually and fermentation by a tempeh fungus was not performed well. Therefore, tempeh which was not hard so that it was broken and did not maintain the shape was completed.

From the above results, when fermentation is performed at a relative humidity of 60 to 78%, it was confirmed that good-quality tempeh which is hard and has good texture and a low microbial level may be produced efficiently .

**[Table 2]**

| Fermentation humidity | Common bacteria (CFU/g) |
|---|---|
| 60% | 1.0.E+08 |
| 68% | 1.6.E+08 |
| 76% | 1.9.E+08 |

### 1-3. Evaluation of quality of wheat tempeh depending on tempeh fermentation temperature

Wheat tempeh was produced in the same manner as in Example 1, except that the fermentation temperature during tempeh fermentation was changed was to 27°C, 30°C, and 33°C. Bacillus cereus which is food poisoning bacteria was counted in the produced wheat tempeh and microbial levels were compared.

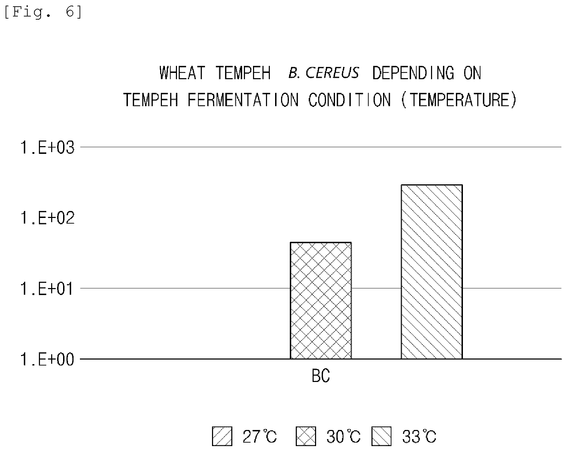

As a result, as shown in the following Table 3 and FIG. 6, it was confirmed that the growth of a tempeh fungus was all smoothly done in a temperature conditions of 27°C to 33°C, and in particular, at 27°C, the level of the food poisoning bacteria was the lowest.

**[Table 3]**

| Fermentation temperature | B. Cereus (CFU/g) |
|---|---|
| 27°C | 0.E+00 |
| 30°C | 5. E+01 |
| 33°C | 4.E+02 |

### 1-4. Evaluation of quality of wheat tempeh depending on moisture content

Wheat tempeh was produced with an amount of a tempeh starter added of 4 wt% or 6 wt% in the same manner as in Example 1, and the moisture content of the completed tempeh was measured. The total bacterial counts (CFU/g) of *Bacillus cereus* which is food poisoning bacteria and common bacteria were measured and microbial levels were compared.

As a result, as shown in the following Table 4, the moisture content of the final wheat tempeh was measured as about 30 to 36 wt%. In addition, as the moisture content included in wheat tempeh is lower, the food poisoning bacteria and the total bacterial count of microorganisms were decreased, and thus, it was confirmed that the growth of a tempeh fungus predominated and a microbial level was lowered.

**[Table 4]**

| Amount (wt%) of tempeh starter added | Moisture content (%) | pH | B. Cereus (CFU/g) | Total bacterial count (CFU/g) |
|---|---|---|---|---|
| 4% | 32.94 | 6.79 | 5.50.E+03 | 5.00.E+08 |
| | 34.94 | 7.01 | 2.50.E+04 | 4.00.E+08 |
| 6% | 30.98 | 6.93 | 5.00.E+02 | 6.00.E+08 |
| | 35.38 | 7.01 | 1. OO.E+03 | 1.05.E+09 |

### 1-5. Evaluation of quality of tempeh depending on raw material grains

Common bacteria, food poisoning bacteria, and fungi in wheat tempeh produced in Example 1 and rice tempeh produced in Example 3 were counted and microbial levels were compared.

As a result, as shown in the following Table 5, the grain tempeh produced by the production method of the present disclosure was all detected to have a common bacterial count of 10⁸ CFU/g or less and a bacillus cereus count of 10³ CFU/g or less, and thus, it was confirmed that the microbial level was lowered. The fungal count was not detected or confirmed to be a low level of 10⁵ CFU/g or less. From the experimental results, it was confirmed that the grain tempeh produced by the production method of the present disclosure was produced to be hard and have good quality and had excellent microbial stability due to smooth growth of a tempeh fungus regardless of the type of grains.

**[Table 5]**

| Classification | Common bacteria | B. Cereus (CFU/g) | Fungus |
|---|---|---|---|
| Wheat tempeh | 1.E+07 | 4.E+02 | N. D |
| Rice tempeh | 2.5.E+07 | N. D | 2.5.E+04 |

### [Experimental Example 2] Evaluation of aged wheat tempeh

The aging degree and pH of aged wheat tempeh produced according to Example 2 were measured.

### 2-1. Evaluation of aging degree of aged wheat tempeh depending on inoculation amount of tempeh fungus

It was confirmed whether the aging degree of an aged product was changed when wheat tempeh produced with different inoculation amounts of a tempeh fungus during production of wheat tempeh was aged.

Specifically, wheat tempeh was produced in the same manner as in Example 1, except that the amount of a tempeh starter added which was inoculated into steamed wheat flour was changed to 3 wt% to 6 wt% with respect to the weight of the wheat flour. Brine having a salinity of 7% was added to the produced wheat tempeh to adjust the moisture content of the mixture to 60% and stirring aging was performed for 14 days to produce an aged product. A formal titration method was used to compare the aging degrees and the pHs of the aged products.

Specifically, the aging degree was evaluated by measuring the amino-type nitrogen content by the following method. First, the aged wheat tempeh was homogenized, a 1% phenolphthalein solution was added to 50 ml of a sample solution diluted 50 times in distilled water, and titration was performed with a 0.1 N sodium hydroxide solution until the color changed to a light red color. 30 ml of a 17.5% formalin solution was titrated by the same method. The aged wheat tempeh solution and the formalin solution were mixed, and then the amino-type nitrogen content (mg%) was measured with the amount (ml) titrated until a light red color was shown with the 0.1 N sodium hydroxide solution. The numerical value of the measured amino-type nitrogen content (mg%) was indicated as an aging degree and evaluated.

As a result, as shown in the following Tables 6 and 7, when the tempeh to which the tempeh starter was added at 3 wt% with respect to the weight of raw material wheat flour was aged, the aged product had a less aging degree and a decreased pH as compared with the case in which tempeh to which the tempeh starter was added at 4 wt% to 6 wt% was aged. From the results as the above, it was confirmed that the aging degree of aged wheat tempeh was adjusted by the aging period and the inoculation amount of tempeh, and when grain tempeh was produced, it was preferred that 4 wt% or more of a tempeh fungus was inoculated.

**[Table 6]**

| Aged wheat tempeh by inoculation amount | Conventional (Ragi 4%) | | Ragi 3% | | Ragi 5% | | Ragi 6% | |
|---|---|---|---|---|---|---|---|---|
| | pH | Aging degree | pH | Aging degree | pH | Aging degree | pH | Aging degree |
| Day 0 | 5.71 | 22.40 | 5.43 | 19.60 | 5.72 | 24.92 | 5.74 | 25.76 |
| Day 1 | 5.41 | 139.72 | 5.20 | 101.36 | 5.37 | 131.04 | 5.39 | 139.72 |
| Day 2 | 5.33 | 194.88 | 5.12 | 156.52 | 5.30 | 184.52 | 5.32 | 191.52 |
| Day 3 | 5.17 | 213.08 | 4.97 | 164.92 | 5.14 | 185.36 | 5.15 | 209.16 |
| Day 4 | 5.27 | 249.48 | 5.07 | 199.08 | 5.24 | 234.92 | 5.24 | 249.20 |
| Day 5 | 5.15 | 212.24 | 4.89 | 159.32 | 5.10 | 219.24 | 5.14 | 210.00 |
| Day 6 | 5.12 | 237.72 | 4.92 | 182.00 | 5.10 | 192.92 | 5.13 | 204.12 |
| Day 7 | 5.07 | 248.08 | 4.91 | 196.56 | 5.07 | 229.60 | 5.08 | 219.24 |

### 2-2. Evaluation of aging degree of aged wheat tempeh depending on aging method

In order to confirm whether the aging degree of aged tempeh was changed by the aging method of wheat tempeh, the aging degrees of aged products depending on stirring during aging were compared.

Specifically, the aging degrees and the pHs of aged wheat tempeh which was stirred and aged as in Example 2 and aged wheat tempeh (control) which was simply settled and aged were compared by the method of the following Experimental Example 2-1.

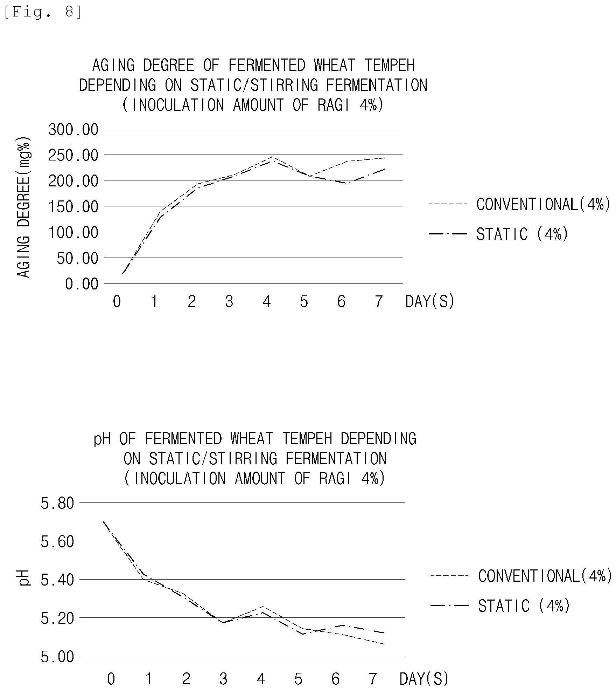

As a result, as shown in the following Table 7 and FIG. 8, it was confirmed that even in the case of tempeh produced by adding the same amount of a tempeh starter, the tempeh which was stirred and aged during aging had an improved aging degree as compared with the tempeh which was settled and aged.

**[Table 7]**

| Aged wheat tempeh depending on aging method | Conventional (Ragi 4%, stirred) | | Static | |
|---|---|---|---|---|
| | pH | Aging degree | pH | Aging degree |
| Day 0 | 5.71 | 22.40 | 5.71 | 22.40 |
| Day 1 | 5.41 | 139.72 | 5.43 | 127.68 |
| Day 2 | 5.33 | 194.88 | 5.32 | 184.80 |
| Day 3 | 5.17 | 213.08 | 5.18 | 210.56 |
| Day 4 | 5.27 | 249.48 | 5.23 | 239.96 |
| Day 5 | 5.15 | 212.24 | 5.12 | 210.28 |
| Day 6 | 5.12 | 237.72 | 5.16 | 198.80 |
| Day 7 | 5.07 | 248.08 | 5.12 | 224.28 |

### 2-3. Evaluation of aging degree of aged wheat tempeh depending on tempeh fermentation humidity

When wheat tempeh produced with different fermentation humidities during wheat tempeh production was aged, it was confirmed whether the aging degree of the aged product was changed depending on the fermentation humidity.

As in Experimental Example 1-2, wheat tempeh was produced with a different fermentation humidity of 60%, 68%, or 76% during tempeh fermentation, the produced wheat tempeh was stirred and aged, and the aging degree and the pH of the aged product were measured in the same manner as in Experimental Example 2-1.

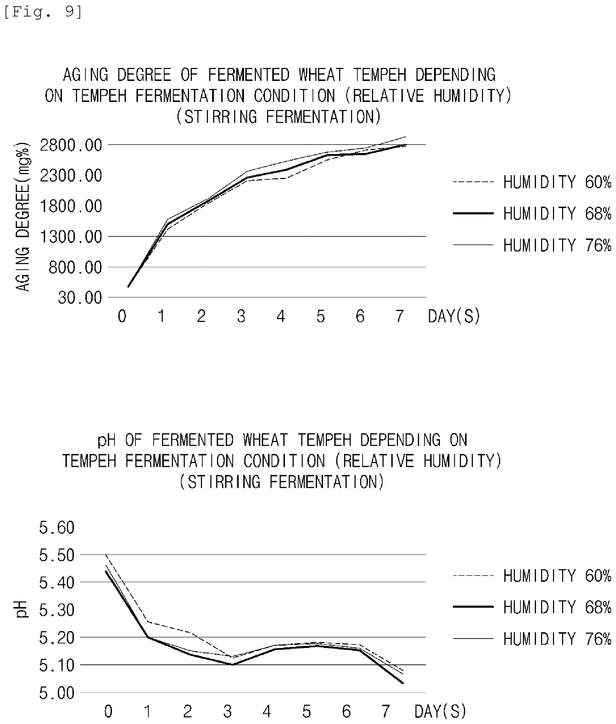

As a result, as shown in the following Table 8 and FIG. 9, it was confirmed that as the relative humidity increased, the common bacterial count was increased. From the results, it was confirmed that the aging degree of the aged wheat tempeh was able to be adjusted depending on the aging period and the fermentation humidity during tempeh production.

**[Table 8]**

| Tempeh fermentatio n condition aged wheat tempeh by (humidity) | Conventional (humidity 60%) | | Humidity: 68% | | Humidity: 76% | |
|---|---|---|---|---|---|---|
| | pH | Aging degree | pH | Aging degree | pH | Aging degree |
| Day 0 | 5.5 0 | 47.60 | 5.4 5 | 46.95 | 5.4 7 | 52.71 |
| Day 1 | 5.2 6 | 145.32 | 5.2 1 | 154.25 | 5.2 1 | 156.80 |
| Day 2 | 5.2 2 | 184.90 | 5.1 4 | 192.05 | 5.1 5 | 191.77 |
| Day 3 | 5.1 2 | 224.11 | 5.1 0 | 231.28 | 5.1 3 | 236.86 |
| Day 4 | 5.1 7 | 228.20 | 5.1 6 | 238.28 | 5.1 7 | 253.40 |
| Day 5 | 5.1 8 | 255.08 | 5.1 7 | 264.04 | 5.1 7 | 269.08 |
| Day 6 | 5.1 7 | 275.52 | 5.1 5 | 266.00 | 5.1 6 | 275.80 |
| Day 7 | 5.0 7 | 277.76 | 5.0 3 | 282.24 | 5.0 6 | 294.28 |

### 2-4. Evaluation of aging degree of aged wheat tempeh depending on fermentation temperature

Wheat tempeh was produced with a different fermentation temperature of 27°C, 30°C, or 33°C during tempeh fermentation, brine having a salinity of 7% was added to the wheat tempeh produced to adjust the moisture content of the mixture to 60%, and the aging degree and the pH of the aged product which was stirred and aged for 14 days were measured.

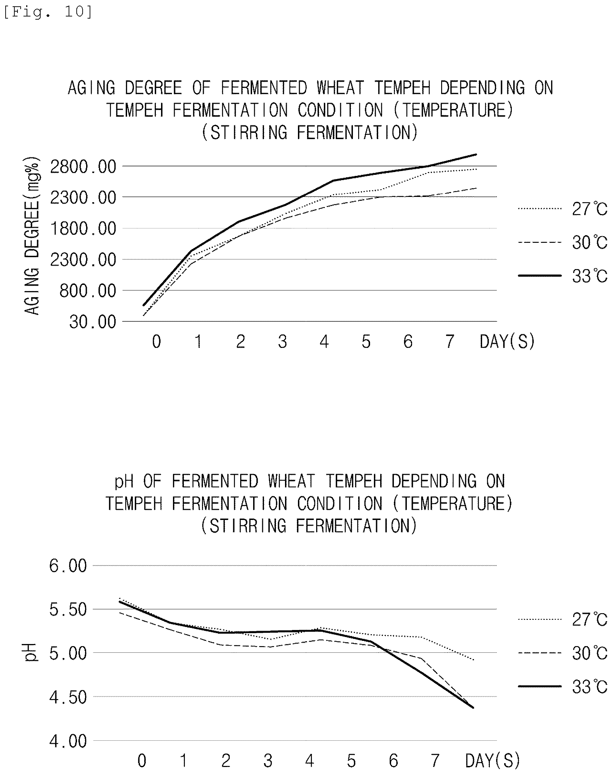

As a result, as shown in the following Table 9 and FIG. 10, it was confirmed that the aging degree was increased due to bacterial growth at a temperature higher than 30°C which is an optimal growth temperature of a tempeh fungus. From the results, it was confirmed that the aging degree of the aged tempeh may be adjusted depending on the aging period and the fermentation temperature during tempeh production.

**[Table 9]**

| Tempeh fermentation condition aged wheat tempeh by (temperature ) | Conventional (temperature 30°C) | | 27°C | | 33°C | |
|---|---|---|---|---|---|---|
| | pH | Aging degree | pH | Aging degree | pH | Aging degree |
| Day 0 | 5.4 7 | 43.96 | 5.6 3 | 42.84 | 5.6 0 | 59.08 |
| Day 1 | 5.3 0 | 125.16 | 5.3 7 | 140.28 | 5.3 7 | 144.76 |
| Day 2 | 5.1 0 | 167.72 | 5.2 7 | 167.16 | 5.2 4 | 188.44 |
| Day 3 | 5.0 8 | 195.16 | 5.1 9 | 201.60 | 5.2 5 | 217.56 |
| Day 4 | 5.1 6 | 215.88 | 5.3 0 | 234.08 | 5.2 8 | 255.64 |
| Day 5 | 5.1 1 | 231.00 | 5.2 2 | 241.92 | 5.1 5 | 268.80 |
| Day 6 | 4.9 5 | 232.40 | 5.1 8 | 269.64 | 4.8 0 | 278.60 |
| Day 7 | 4.4 0 | 245.56 | 4.9 4 | 274.96 | 4.4 0 | 298.20 |

### 2-5. Evaluation of aging degree of aged wheat tempeh depending on moisture content during aging

In order to confirm whether the aging degree of was changed depending on the moisture content of a mixture during wheat tempeh aging, a mixture of wheat tempeh and steamed wheat rice was prepared in the same manner as in Example 2 and aged.

Specifically, steamed wheat rice was added to wheat tempeh, brine having a salinity of 7% was added to adjust the moisture content of the mixture to 50% or 60%, differently, and the mixture was aged to produce an aged mixture of wheat tempeh and wheat rice.

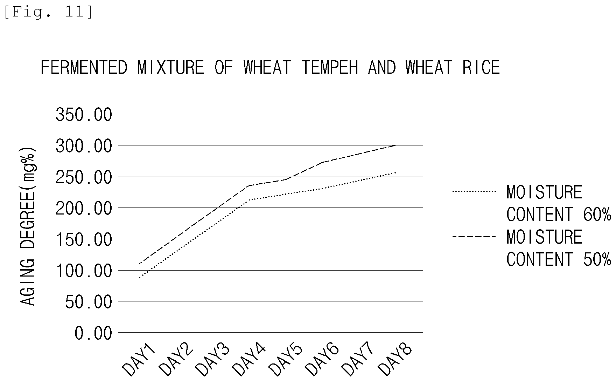

As a result of the experiment, as shown in FIG. 11, when the moisture content of the mixture was 60%, the aging degree of the aged product was improved as compared with the case of having a moisture content of 50% of the mixture. From the results as such, it was confirmed that the aging degree of the aged tempeh was able to be adjusted depending on the aging period and the moisture content included in the aged grain tempeh.

### 2-6. Evaluation of aging degree of aged wheat tempeh depending on addition of extra rice

Steamed wheat rice was added to the aged wheat tempeh as extra rice, mixed, and aged again to produced aged mixture, and the aging degree of the mixture was evaluated.

Specifically, steamed wheat rice was added to the wheat tempeh produced in Example 1, and brine having a salinity of 7% was added to prepare a mixture. At this time, the steamed wheat rice was differently added at 0 to 20 wt% with respect to the total weight of the mixture, the moisture content of the mixture was adjusted to 60%, and then stirred and aged for 14 days to produce an aged product, and the aging degree and the pH thereof were measured.

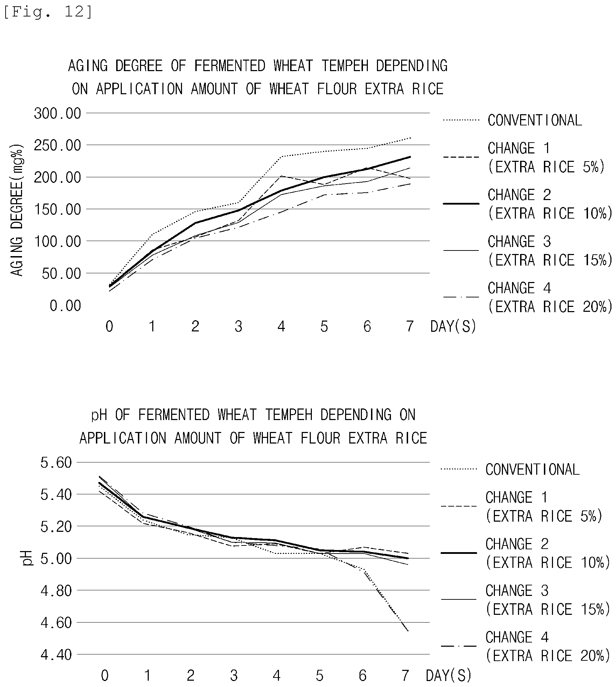

As a result, as shown in the following Table 10 and FIG. 12, as the amount of extra rice was increased, the aging degree was decreased, and thus, it was confirmed that the aging degree of the aged tempeh was able to be adjusted depending on the added amount.

**[Table 10]**

| | Conventional (extra rice 0%) | | Extra rice 5% | | Extra rice 10% | | Extra rice 15% | | Extra rice 20% | |
|---|---|---|---|---|---|---|---|---|---|---|
| | pH | Aging degree | pH | Aging degree | pH | Aging degree | pH | Aging degree | pH | Aging degree |
| Day 0 | 5.45 | 29.40 | 5.42 | 28.56 | 5.47 | 27.44 | 5.51 | 26.32 | 5.51 | 21.56 |
| Day 1 | 5.23 | 108.64 | 5.22 | 85.12 | 5.26 | 83.16 | 5.26 | 77.00 | 5.28 | 70.56 |
| Day 2 | 5.15 | 145.88 | 5.16 | 105.28 | 5.19 | 127.12 | 5.20 | 108.08 | 5.20 | 104.44 |
| Day 3 | 5.13 | 159.32 | 5.08 | 131.32 | 5.13 | 147.84 | 5.10 | 127.68 | 5.10 | 120.12 |
| Day 4 | 5.03 | 230.72 | 5.09 | 199.64 | 5.11 | 176.40 | 5.09 | 171.92 | 5.08 | 143.92 |
| Day 5 | 5.03 | 238.84 | 5.03 | 187.60 | 5.05 | 199.64 | 5.03 | 185.64 | 5.06 | 171.64 |
| Day 6 | 4.93 | 244.44 | 5.07 | 215.88 | 5.04 | 211.40 | 5.03 | 193.20 | 4.91 | 175.00 |
| Day 7 | 4.56 | 259.84 | 5.03 | 199.64 | 5.00 | 229.88 | 4.96 | 213.08 | 4.55 | 187.04 |

### 2-7. Evaluation of aging degree of aged tempeh depending on raw material grains

In order to confirm whether the aging degree of aged tempeh was changed depending on the type of raw material grains, brine having a salinity of 7% was added to each of the wheat tempeh produced in Example 1 and rice tempeh produced in Example 3 so that the moisture content was about 60%, and aging was performed while stirring was performed at a speed of 50 to 300 rpm at room temperature, and the aging degrees of the produced aged products were compared.

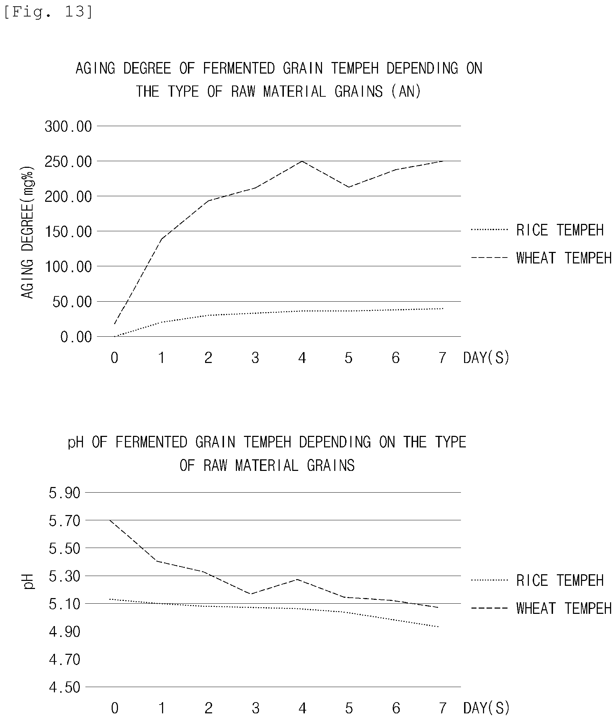

As a result, as shown in the following Table 11 and FIG. 13, it was confirmed that rice tempeh had a low aging degree and a low increase rate thereof as compared with the aged wheat tempeh by the low protein source content. It was confirmed that aged tempeh was able to be produced using various grains.

**[Table 11]**

| | Rice tempeh | | Wheat tempeh | |
|---|---|---|---|---|
| | pH | Aging degree | pH | Aging degree |
| Day 0 | 5.12 | 0.00 | 5.71 | 22.40 |
| Day 1 | 5.10 | 21.56 | 5.41 | 139.72 |
| Day 2 | 5.08 | 31.08 | 5.33 | 194.88 |
| Day 3 | - | - | 5.17 | 213.08 |
| Day 4 | 5.06 | 35.00 | 5.27 | 249.48 |
| Day 5 | 5.04 | 34.72 | 5.15 | 212.24 |
| Day 6 | - | - | 5.12 | 237.72 |
| Day 7 | 4.94 | 38.92 | 5.07 | 248.08 |

### [Production Example] Production of sauce including wheat tempeh or aged wheat tempeh

3 wt% to 50 wt% of wheat tempeh produced in Example 1 was added depending on the taste direction of the sauce to be desired, chili pepper powder, chili pepper, salt, soy sauce, tomato ketchup, and also, a composite seasoning food (for example, chili pepper seasoning), sugars (sugar, high fructose, sugar syrup, oligosaccharide, starch syrup), flavoring enhancer, and the like were mixed, sterilized at 70°C to 110°C for 10 minutes to 1 hour, cooled, and packaged to produce a wheat tempeh-containing sauce.

In addition, 3 wt% to 50 wt% of aged wheat tempeh or an aged mixture of wheat tempeh and wheat rice produced in Example 2 was added depending on the taste direction of the sauce to be desired, chili pepper powder, chili pepper, salt, soy sauce, tomato ketchup, and also, a composite seasoning food (for example, chili pepper seasoning), sugars (sugar, high fructose, sugar syrup, oligosaccharide, starch syrup), flavoring enhancer, and the like were mixed, sterilized at 70°C to 110°C for 10 minutes to 1 hour, cooled, and packaged to produce a sauce including aged wheat tempeh or an aged mixture of wheat tempeh and wheat rice.

## Claims

1. A method of producing grain tempeh, the method comprising:
(a) steaming grains;
(b) inoculating a tempeh fungus into the steamed grains; and
(c) fermenting the steamed grains inoculated with the tempeh fungus.

2. The method of producing grain tempeh of claim 1, wherein the grains are any one or more selected from the group consisting of wheat, wheat rice, rye, rice, barley, foxtail millet, oats, proso millet, sorghum, and corn.

3. The method of producing grain tempeh of claim 1 or claim 2, wherein the steaming of grains in (a) is performed by a continuous steaming method or a steam pressurization method.

4. The method of producing grain tempeh of any one of claims 1 to 3, wherein (b) is adding a tempeh starter at 2 wt% to 8 wt% with respect to a total weight of the steamed grains.

5. The method of producing grain tempeh of any one of claims 1 to 4, wherein the fermenting in (c) is performed at a temperature of 25°C to 35°C.

6. The method of producing grain tempeh of any one of claims 1 to 5, wherein the fermenting in (c) is performed at a humidity of 60% to 80%.

7. Grain tempeh produced by the method of producing grain tempeh of any one of claims 1 to 6.

8. A sauce comprising the grain tempeh of claim 7.

9. A method of producing aged grain tempeh, the method comprising:
(a) producing grain tempeh by the method of producing grain tempeh of any one of claims 1 to 6; and
(b) aging a mixture in which at least one or more of saline and brine is added to the grain tempeh.

10. The method of producing aged grain tempeh of claim 9, further comprising adding one or more of grains and steamed grains to the mixture in the aging.

11. The method of producing aged grain tempeh of claim 9 or claim 10, wherein a moisture content included in the mixture is 40% to 70% in the aging.

12. Aged grain tempeh produced by the method of any one of claims 9 to 11.

13. A sauce comprising the aged grain tempeh of claim 12.
